# EUROPEAN PATENT APPLICATION

(11) **EP 4 692 794 A1**
(43) Date of publication of application: **11.02.2026**
(21) Application number: 24780808.2
(22) Date of filing: 29.03.2024
(51) Int. Cl.: G01N 33/531

(54) **COMPOSITION, AQUEOUS SOLUTION OF ENDOCRINE PROTEIN, ADSORPTION INHIBITOR, METHOD FOR INHIBITING ADSORPTION OF ENDOCRINE PROTEIN, METHOD FOR MEASURING AMOUNT OF ENDOCRINE PROTEIN, AND REAGENT KIT FOR MEASURING ENDOCRINE PROTEIN**

(30) Priority: 30.03.2023 JP 2023055296
(71) Applicant: FUJIFILM Corporation, Tokyo 106-8620 (JP)
(72) Inventor: SAITO, Yumi, Tokyo 106-8620 (JP); EBISU, Yu, Tokyo 106-8620 (JP)
(74) Representative: Dehns Germany Partnerschaft mbB
(86) International application number: PCT/JP2024/013046
(87) International publication number: WO 2024/204713

(57) **Abstract**

An object of the present invention is to provide a composition in which adsorption of an endocrine protein to a container is suppressed in a solution obtained by dissolving the composition in a solvent, an aqueous solution of an endocrine protein in which adsorption of an endocrine protein to a container is suppressed, an adsorption inhibitor in which adsorption of an endocrine protein to a container is suppressed, a method for suppressing adsorption, a method for measuring an amount of an endocrine protein using the composition or the aqueous solution as a standard sample, and a kit including the composition or the aqueous solution. According to the present invention, there are provided a composition containing an endocrine protein and at least two compounds selected from the group consisting of a fluorine-containing surfactant, a collagen peptide, and a milk protein; an aqueous solution containing water, an endocrine protein, and at least two compounds selected from the group consisting of the above-described surfactant, a collagen peptide, and a milk protein; an adsorption inhibitor; a method for suppressing adsorption; a method for measuring an amount of an endocrine protein; and a kit.

## Description

### Technical Field

The present invention relates to a composition, an aqueous solution of an endocrine protein, an adsorption inhibitor, a method for suppressing adsorption of an endocrine protein, a method for measuring an amount of an endocrine protein, and a reagent kit for measuring an endocrine protein.

### Background Art

Currently, a composition containing an endocrine protein or an aqueous solution containing an endocrine protein is used in various fields.

In these compositions or aqueous solutions, there is a need to strictly control the amount of the contained endocrine protein.

The above composition or aqueous solution may be used, for example, as a standard product for creating a calibration curve for converting a measured value into a content, a calibration sample for calibrating the calibration curve, or a quality control sample for confirming that the calibration is correctly performed, for the purpose of quantifying the content of the endocrine protein in the sample.

In any of these cases, the above-described composition or aqueous solution in which the content (content concentration) of the endocrine protein is known is used. In these cases, in a case where the content of the above-described endocrine protein fluctuates during storage, preparation, or the like, a quantitative result may not be correctly obtained.

For example, Patent Document 1 describes an immunoassay reagent (M) containing an immunoassay solid-phase carrier (E), a sugar (A), and water, in which a weight proportion of the water is 20% to 60% by weight with respect to a weight of the immunoassay reagent.

### Prior Art Documents

### Patent Documents

Patent Document 1: JP2021-071476A

### Summary of Invention

### Object to be solved by the invention

An object of the present invention is to provide a composition in which adsorption of an endocrine protein to a container is suppressed in a solution obtained by dissolving the composition in a solvent.

In addition, another object of the present invention is to provide an aqueous solution of an endocrine protein in which adsorption of the endocrine protein to a container is suppressed.

Furthermore, another object of the present invention is to provide an adsorption inhibitor in which adsorption of an endocrine protein to a container is suppressed, a method for suppressing adsorption, a method for measuring an amount of an endocrine protein using the composition or the aqueous solution as a standard sample, and a kit including the composition or the aqueous solution.

### Means for solving the object

Examples of representative embodiments according to the present invention are described below.
<1> A composition comprising:
   an endocrine protein; and
   at least two compounds selected from the group consisting of a fluorine-containing surfactant, a collagen peptide, and a milk protein.
<2> The composition according to <1>, in which the composition is a standard sample for measuring an amount of an endocrine protein.
<3> The composition according to <1> or <2>, in which the composition is contained in a container.
<4> The composition according to <3>, in which a material of the container is glass or plastic.
<5> The composition according to any one of <1> to <4>, in which a content of the fluorine-containing surfactant is 0.1% to 1% by mass with respect to a total solid content of the composition.
<6> The composition according to any one of <1> to <5>, in which a content of the collagen peptide is 1% to 30% by mass with respect to a total solid content of the composition.
<7> The composition according to any one of <1> to <6>, in which a content of the milk protein is 0.1% to 10% by mass with respect to a total solid content of the composition.
<8> The composition according to any one of <1> to <7>, in which the endocrine protein is luteinizing hormone, prolactin, or human chorionic gonadotropin.
<9> An aqueous solution of an endocrine protein, comprising:
   water;
   an endocrine protein; and
   at least two compounds selected from the group consisting of a fluorine-containing surfactant, a collagen peptide, and a milk protein.
<10> The aqueous solution according to <9>, in which the aqueous solution is a standard sample for measuring an amount of an endocrine protein.
<11> The aqueous solution according to <9> or <10>, in which the aqueous solution is contained in a container.
<12> The aqueous solution according to <11>, in which a material of the container is glass or plastic.
<13> The aqueous solution according to any one of <9> to <12>, in which a content of the fluorine-containing surfactant is 0.1% to 1% by mass with respect to a total solid content of the aqueous solution.
<14> The aqueous solution according to any one of <9> to <13>, in which a content of the collagen peptide is 1% to 30% by mass with respect to a total solid content of the aqueous solution.
<15> The aqueous solution according to any one of <9> to <14>, in which a content of the milk protein is 0.1% to 10% by mass with respect to a total solid content of the aqueous solution.
<16> The aqueous solution according to any one of <9> to <15>, in which the endocrine protein is luteinizing hormone, prolactin, or human chorionic gonadotropin.
<17> An adsorption inhibitor for an endocrine protein, comprising:
   at least two compounds selected from the group consisting of a fluorine-containing surfactant, a collagen peptide, and a milk protein.
<18> A method for suppressing adsorption of an endocrine protein, the method comprising:
   bringing a glass or plastic member into contact with an endocrine protein in presence of water and at least two compounds selected from the group consisting of a fluorine-containing surfactant, a collagen peptide, and a milk protein.
<19> A method for suppressing adsorption of an endocrine protein, comprising:
   bringing a glass or plastic member into contact with an aqueous solution containing water, an endocrine protein, and at least two compounds selected from the group consisting of a fluorine-containing surfactant, a collagen peptide, and a milk protein.
<20> A method for measuring an amount of an endocrine protein, the method comprising:
   using, as a standard sample, the composition according to any one of <1> to <8> or the aqueous solution according to any one of <9> to <16>.
<21> A reagent kit for measuring an endocrine protein, the reagent kit comprising:
   the composition according to any one of <1> to <8> or the aqueous solution according to any one of <9> to <16>.

### Effect of the invention

According to the present invention, there is provided a composition in which adsorption of an endocrine protein to a container is suppressed in a solution obtained by dissolving the composition in a solvent.

In addition, according to the present invention, an aqueous solution of an endocrine protein in which adsorption of the endocrine protein to a container is suppressed is provided.

Furthermore, according to the present invention, there are provided an adsorption inhibitor in which adsorption of an endocrine protein to a container is suppressed, a method for suppressing adsorption, a method for measuring an amount of an endocrine protein using the composition or the aqueous solution as a standard sample, and a kit including the composition or the aqueous solution.

### Embodiments for carrying out the invention

Hereinafter, the main embodiments according to the present invention will be described. However, the present invention is not limited to the specified embodiments.

In the present specification, a numerical value range described by using "to" means a range including numerical values described before and after the preposition "to" as a lower limit value and an upper limit value, respectively.

In the present specification, the total solid content refers to the total mass of components excluding a solvent from the entire components of the composition. In addition, in the present specification, the concentration of solid contents is a mass percentage of other components excluding a solvent with respect to the total mass of the composition.

In the present specification, the unit M indicates mol/L. Similarly, mM indicates mmol/L, and µM indicates µmol/L.

In the present specification, the unit N refers to a value obtained by multiplying a molar concentration by the valence of an acid or an alkali.

In the present specification, unless otherwise specified, the temperature is 23°C, the atmospheric pressure is 101,325 Pa (1 atm), and the relative humidity is 50%RH.

In addition, in the present specification, a combination of preferred aspects is a more preferred aspect.

### (Composition)

The composition according to the embodiment of the present invention contains an endocrine protein, and at least two compounds selected from the group consisting of a fluorine-containing surfactant, a collagen peptide, and a milk protein.

Among these, from the viewpoint of suppressing adsorption of the endocrine protein to the container, the composition according to the embodiment of the present invention preferably contains at least a collagen peptide and a milk protein.

In a case where the composition according to the embodiment of the present invention contains at least a collagen peptide and a milk protein, an effect of suppressing the adsorption for various endocrine proteins may be obtained.

According to the composition according to the embodiment of the present invention, the adsorption of the endocrine protein to the container in a dissolved solution obtained by dissolving the composition in a solvent is suppressed.

The present inventors have found that at least two compounds selected from the group consisting of a fluorine-containing surfactant, a collagen peptide, and a milk protein work synergistically to suppress adsorption of an endocrine protein to a container, thereby completing the present invention.

### <Endocrine protein>

The endocrine protein is not particularly limited, and is preferably luteinizing hormone (LH), prolactin, or human chorionic gonadotropin (hCG).

In a case of these endocrine proteins, the effect of suppressing adsorption is easily obtained by the present invention.

### <Container>

It is preferable that the composition according to the embodiment of the present invention is contained in a container.

The material of the container (the material of the member in contact with the composition) is preferably glass or plastic.

The plastic is not particularly limited, and examples thereof include polyethylene (PE), polypropylene (PP), polystyrene (PS), polyvinyl chloride (PVC), polyurethane (PU), polytetrafluoroethylene (PTFE), polyethylene terephthalate (PET), an acrylic resin such as polymethyl methacrylate (PMMA), and a copolymer of polyethylene and polypropylene, where polyethylene (PE), polypropylene (PP), or polystyrene (PS) is preferable.

The shape of the container is not particularly limited, and examples thereof include containers such as a vial, a tube (a microtube), a test tube, a microtiter plate (an ELISA plate), a microarray substrate, a tray, or a cell.

The container may have a lid. The shape of the lid of the container is not particularly limited as long as the opening of the container is closed. Examples of the lid of the container include a lid having a surface composed of rubber (a rubber lid) and a lid having a surface composed of plastic (a plastic lid).

### <Fluorine-containing surfactant>

The fluorine-containing surfactant may be any of a nonionic surfactant, an anionic surfactant, a cationic surfactant, or an amphoteric surfactant, but a nonionic surfactant is preferable.

In the fluorine-containing surfactant, it is preferable that 30 mol% or more of the hydrogen atoms capable of being substituted with fluorine atoms in one molecule are substituted with fluorine atoms, it is more preferable that 40 mol% or more of the hydrogen atoms capable of being substituted with fluorine atoms in one molecule are substituted with fluorine atoms, and it is still more preferable that 50 mol% or more of the hydrogen atoms capable of being substituted with fluorine atoms in one molecule are substituted with fluorine atoms. The upper limit of the above-described proportion is not particularly limited, and may be 100% by mole.

The fluorine-containing surfactant is preferably a compound having a perfluoroalkyl group or a perfluoroalkenyl structure, and more preferably a compound having a perfluoroalkenyl structure.

The above-described fluorinated alkyl group and fluorinated alkenyl group may have any structure represented by a linear, branched, or cyclic structure or a combination thereof, but preferably have a branched structure.

For example, a surfactant having a fluorinated alkenyl group represented by Formula (F) is preferable. In Formula (F), * represents a bonding site to another structure.

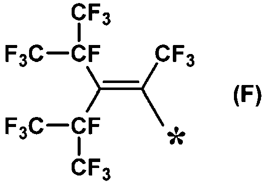

Examples of a commercially available product of the fluorine-containing surfactant include: Megaface F-171, F-172, F-173, F-176, F-177, F-141, F-142, F-143, F-144, F-437, F-475, F-477, F-479, F-482, F-551-A, F-552, F-554, F-555-A, F-556, F-557, F-558, F-559, F-560, F-561, F-565, F-563, F-568, F-575, F-780, EXP.MFS-330, EXP.MFS-578, EXP.MFS-579, EXP.MFS-586, EXP.MFS-587, EXP.MFS-628, EXP.MFS-631, EXP.MFS-603, R-41, R-41-LM, R-01, R-40, R-40-LM, RS-43, TF-1956, RS-90, R-94, RS-72-K, and DS-21 (all of which are manufactured by DIC Corporation); Fluorad FC430, FC431, and FC171 (all of which are manufactured by Sumitomo 3M Ltd.); Surflon S-382, SC-101, SC-103, SC-104, SC-105, SC-1068, SC-381, SC-383, S-393, and KH-40 (all of which are manufactured by AGC Inc.); and Polyfox PF636, PF656, PF6320, PF6520, and PF7002 (all of which are manufactured by OMNOVA Solutions Inc.); Ftergent 710FL, 710FM, 610FM, 601AD, 601ADH2, 602A, 215M, 245F, 251, 212M, 250, 209F, 222F, 208G, 710LA, 710FS, 730LM, 650AC, 681, and 683 (all of which are manufactured by NEOS COMPANY LIMITED); U-120E (Uni-chem Co., Ltd.); and the like.

In a case where the composition according to the embodiment of the present invention contains a fluorine-containing surfactant, the content of the fluorine-containing surfactant is preferably 0.1% to 1% by mass, more preferably 0.15% to 0.9% by mass, and still more preferably 0.2% to 0.8% by mass with respect to the total solid content of the composition.

The composition according to the embodiment of the present invention may contain two or more types of fluorine-containing surfactants. In a case where two or more types thereof are contained, the total content thereof is preferably within the above-described range.

### <Collagen peptide>

The collagen peptide is obtained, for example, by directly hydrolyzing collagen or hydrolyzing collagen through gelatin with an enzyme or an acid. The molecular weight of the collagen peptide can be adjusted depending on the conditions of hydrolysis.

The collagen peptide may be derived from any type of collagen, and the type of collagen is not particularly limited. As the collagen peptide, for example, a peptide derived from collagen of any type of type I to type XIII can be used.

In addition, the collagen peptide may be chemically synthesized.

The number-average molecular weight of the collagen peptide is preferably 500 to 10,000, more preferably 1,000 to 6,000, and still more preferably 1,500 to 5,000.

The "number-average molecular weight of collagen peptide" refers to a value of a number-average molecular weight (Mn) measured by gel permeation chromatography (GPC: protein standard). It is noted that in a case where a commercially available product is used, product information provided from a supply source may be followed.

In a case where the composition according to the embodiment of the present invention contains a collagen peptide, the content of the collagen peptide is preferably 1% to 30% by mass, more preferably 2% to 20% by mass, and still more preferably 3% to 15% by mass with respect to the total solid content of the composition.

The composition according to the embodiment of the present invention may contain two or more types of collagen peptides. In a case where two or more types thereof are contained, the total content thereof is preferably within the above-described range.

### <Milk protein>

As the milk protein, casein, a concentrated whey protein, non-fat dry milk, skim milk, or the like can be used.

Among these, it is preferable that the milk protein contains at least casein.

In a case where a milk protein is used, the milk protein and a buffer agent may be used in combination. As the buffer agent, a known buffer agent can be used without particular limitation, and examples thereof include an organic acid buffer agent.

For example, a powder in which the milk protein and the buffer agent are mixed in advance can also be contained in the composition. Examples of such a product include BLOCK ACE (manufactured by KAC Co., Ltd.).

In a case where the composition according to the embodiment of the present invention contains a milk protein, the content of the milk protein is preferably 0.1% to 10% by mass, more preferably 0.5% to 5% by mass, and still more preferably 1% to 4% by mass with respect to the total solid content of the composition.

The composition according to the embodiment of the present invention may contain two or more types of milk proteins. In a case where two or more types thereof are contained, the total content thereof is preferably within the above-described range.

### <Other components>

The composition according to the embodiment of the present invention may further contain other components.

Examples of the other components include a buffering agent, another adsorption inhibitor, a preservative (for example, sodium azide, salicylic acid, benzoic acid, or the like), an excipient, and a reaction accelerator.

Examples of the buffer agents include N-(2-acetoamido)-2-aminoethanesulfonic acid (ACES), N-(2-acetoamido)iminodiacetic acid (ADA), N,N-bis(2-hydroxyethyl)-2-aminoethanesulfonic acid (BES), N,N-bis(2-hydroxyethyl)glycine (Bicine), bis(2-hydroxyethyl)iminotris(hydroxyethyl)methane (Bis-Tris), N-cyclohexyl-3-aminopropanesulfonic acid (CAPS), N-cyclohexyl-2-hydroxy-3-aminopropanesulfonic acid (CAPSO), N-cyclohexyl-2-aminoethanesulfonic acid (CHES), 3-[N,N-bis(2-hydroxyethyl)amino]-2-hydroxypropanesulfonic acid (DIPSO), 3-[4-(2-hydroxyethyl)-1-piperazinyl]propanesulfonic acid (EPPS), 2-[4-(2-hydroxyethyl)-1-piperazinyl]ethanesulfonic acid (HEPES), 2-hydroxy-3-[4-(2-hydroxyethyl)-1-piperazinyl]propanesulfonic acid (HEPPSO), 2-(N-morpholino)ethanesulfonic acid (MES), 3-(N-morpholino)propanesulfonic acid (MOPS), 2-hydroxy-3-(N-morpholino)propanesulfonic acid (MOPSO), piperazine-1,4-bis(2-ethanesulfonic acid) (PIPES), piperazine-1,4-bis(2-hydroxy-3-propanesulfonic acid) (POPSO), N-tris(hydroxymethyl)methyl-3-aminopropanesulfonic acid (TAPS), 2-hydroxy-N-tris(hydroxymethyl)methyl-3-aminopropanesulfonic acid (TAPSO), N-tris(hydroxymethyl)methyl-2-aminoethanesulfonic acid (TES), N-[tris(hydroxymethyl)methyl]glycine (Tricine), phosphates, acetates, citrates, tris(hydroxymethyl)aminomethane, and the like.

Examples of the other adsorption inhibitors include a surfactant that does not correspond to the above-described fluorine-containing surfactant, and a peptide adsorption inhibitor such as albumin (for example, bovine serum albumin).

### <Solvent>

It is preferable that the composition according to the embodiment of the present invention does not substantially contain water.

In the present specification, the case where the composition does not substantially contain water means that the content of water is 1% by mass or less with respect to the total mass of the composition, and the content of water is preferably 0.1% by mass or less, more preferably 0.05% by mass or less, and still more preferably 0.01% by mass or less.

In addition, the composition according to the embodiment of the present invention preferably does not substantially contain a solvent other than water.

Examples of the solvent other than water include an organic solvent such as an alcohol.

In the present specification, the case where the composition does not substantially contain a solvent other than water means that the content of the solvent other than water is 1% by mass or less with respect to the total mass of the composition, and the content of the solvent other than water is preferably 0.1% by mass or less, more preferably 0.05% by mass or less, and still more preferably 0.01% by mass or less.

### <Standard sample>

The composition according to the embodiment of the present invention is preferably a standard sample for measuring the amount of the endocrine protein.

The standard sample is used for quantifying an endocrine protein by, for example, an analytical method such as an immunological measurement method or a mass spectrometric method. Specifically, the composition according to the embodiment of the present invention is used as a calibrator, a control, or the like for creating a calibration curve showing a correlation between a known amount (concentration) of an endocrine protein contained in the above-described standard sample and a measured value obtained by various analytical methods, confirming the effectiveness of accuracy control or calibration of an analytical instrument, and investigating the stability of quantitative analysis over time. Examples of the measured value include an absorbance, an amount of change in absorbance, a transmitted light intensity, an amount of change in transmitted light intensity, a fluorescence intensity, an amount of change in fluorescence intensity, a luminescence intensity, an amount of change in luminescence intensity, a turbidity, a rate of change in turbidity, a scattered light intensity, a rate of change in scattered light, a reflectivity, an amount of change in reflectivity, a refractive index, and an amount of change in refractive index.

Here, it is preferable that the standard sample is any one of a calibrator, a control, or a standard product.

In the present specification, the calibrator refers to a sample in which the amount of the endocrine protein is known, and is a calibration sample for correcting and calibrating the differences between measurement devices and the influence of storage in a case of measuring the amount of the endocrine protein.

In the present specification, the control refers to a sample in which the amount of the endocrine protein is known, and is a sample for accuracy control for confirming whether or not the calibration with the calibration sample or the like has been correctly performed.

In the present specification, the standard product is a sample for creating a calibration curve used to create a calibration curve in a measurement system. For example, in the case of an automatic measuring device, a calibration curve is created at the time of factory shipment, and in a case of measurement such as an ELISA method, a calibration curve is created for each measurement.

In such an application, in a case where the concentration of certain amount of the endocrine protein in the standard sample is changed by adsorption to a container or the like, the creation of a calibration curve, accuracy control, calibration, and the like are not correctly performed. As a result, in a case of changing the absorbance, the luminescence intensity, the fluorescence intensity, and the like, which are the measurement results, to the concentration, a concentration different from the actual concentration of the endocrine protein in the sample is calculated.

According to the composition according to the embodiment of the present invention, since adsorption to the container or the like is suppressed, the composition according to the embodiment of the present invention is considered to be extremely useful as a standard sample.

### <Method for producing composition>

The composition according to the embodiment of the present invention can be obtained, for example, by dissolving an endocrine protein and at least two compounds selected from the group consisting of a fluorine-containing surfactant, a collagen peptide, and a milk protein in a solvent such as water to prepare a solution, and then drying the solution by a method such as freeze-drying.

The above-described solution can further contain the above-described other components.

The drying is not particularly limited and can be performed by a known method. For example, the drying method may be determined in consideration of denaturation of an endocrine protein, a collagen peptide, a milk protein, and the like, and decomposition of a fluorine-containing surfactant.

### (Aqueous solution of endocrine protein)

The aqueous solution of the endocrine protein according to the embodiment of the present invention (hereinafter, also simply referred to as "aqueous solution of the embodiment of the present invention") contains water, an endocrine protein, and at least two compounds selected from the group consisting of a fluorine-containing surfactant, a collagen peptide, and a milk protein.

The components to be contained, the content, the container, and the application of the aqueous solution of the endocrine protein according to the embodiment of the present invention is the same as that of the composition according to the embodiment of the present invention except that the endocrine protein is dissolved in the water, and the preferred aspects are also the same.

The content of water in the aqueous solution according to the embodiment of the present invention is preferably 50% to 99% by mass, more preferably 60% to 99% by mass, and still more preferably 70% to 99% by mass, with respect to the total mass of the aqueous solution.

The aqueous solution according to the embodiment of the present invention may further contain a solvent other than water.

Examples of the solvent other than water include an organic solvent such as an alcohol.

In addition, an aspect in which the aqueous solution according to the embodiment of the present invention does not substantially contain a solvent other than water is also one of the preferred aspects of the present invention.

The aqueous solution according to the embodiment of the present invention can be adjusted by dissolving each component contained in the aqueous solution according to the embodiment of the present invention in water (as necessary, a solvent other than water may be further contained).

Here, the aqueous solution according to the embodiment of the present invention may be prepared by dissolving, in water, the composition according to the embodiment of the present invention, which is in an aspect, in which it does not substantially contain water such as a freeze-dried product.

### (Adsorption inhibitor)

The adsorption inhibitor according to the embodiment of the present invention contains at least two compounds selected from the group consisting of a fluorine-containing surfactant, a collagen peptide, and a milk protein.

In the adsorption inhibitor according to the embodiment of the present invention, the preferred aspects of the fluorine-containing surfactant, the collagen peptide, and the milk protein are the same as the preferred aspects of these compounds in the composition according to the embodiment of the present invention.

### (Method for suppressing adsorption of endocrine protein)

A first aspect of a method for suppressing adsorption of an endocrine protein according to the embodiment of the present invention includes bringing a glass or plastic member into contact with an endocrine protein in the presence of water and at least two compounds selected from the group consisting of a fluorine-containing surfactant, a collagen peptide, and a milk protein.

Examples of such an aspect include an aspect in which the endocrine protein is dissolved in the solution in the container in a state in which the solution containing at least water and at least two compounds selected from the group consisting of a fluorine-containing surfactant, a collagen peptide, and a milk protein is placed in the container.

Examples of another aspect include an aspect in which an aqueous solution of the composition according to the embodiment of the present invention is prepared by adding a solvent containing water to a container containing the composition according to the embodiment of the present invention.

In addition, a second aspect of the method for suppressing adsorption of an endocrine protein according to the present invention includes bringing a glass or plastic member into contact with an aqueous solution containing water, an endocrine protein, and at least two compounds selected from the group consisting of a fluorine-containing surfactant, a collagen peptide, and a milk protein.

The aqueous solution in the second aspect is preferably the aqueous solution according to the embodiment of the present invention.

The above-described member is not particularly limited, and examples thereof include containers such as a vial bottle, a tube (microtube), a test tube, a microtiter plate (ELISA plate), a microarray substrate, a tray, and a cell; a lid of a container such as a stopper and a cap; beads, particles, a pipette, a chip, a disk-shaped piece, and the like. It is not necessary that all of these members are composed of glass or plastic, and a part of a surface of these members, which comes into contact with the aqueous solution, may be glass or plastic.

Examples of the second aspect include an aspect in which the aqueous solution according to the embodiment of the present invention is transferred to a glass or plastic container; an aspect in which the aqueous solution according to the embodiment of the present invention is acquired and held with a glass or plastic pipette; and an aspect in which the aqueous solution according to the embodiment of the present invention is brought into contact with glass or plastic beads, particles, or the like.

Hereinafter, the first aspect and the second aspect of the method for suppressing adsorption of an endocrine protein according to the embodiment of the present invention will be collectively referred to as "the method for suppressing adsorption according to the embodiment of the present invention".

**In** the method for suppressing adsorption according to the embodiment of the present invention, the preferred aspects of the endocrine protein, the fluorine-containing surfactant, the collagen peptide, and the milk protein are the same as the preferred aspects of these compounds in the above-described composition according to the embodiment of the present invention.

### (Method for measuring amount of endocrine protein)

The method for measuring the amount of the endocrine protein according to the embodiment of the present invention includes using the composition according to the embodiment of the present invention or the aqueous solution according to the embodiment of the present invention as a standard sample.

By using the composition according to the embodiment of the present invention or the aqueous solution according to the embodiment of the present invention as a standard sample, the adsorption of the endocrine protein is suppressed, and more accurate measurement is possible.

The measuring method for the amount of the endocrine protein according to the embodiment of the present invention can be performed by, for example, an immunological measuring method.

The immunological measuring method may be any method as long as it is a method of performing an antigen-antibody reaction using an anti-endocrine protein antibody. The measurement principle of the immunological measuring method is not particularly limited, and examples of the immunological measuring method include a sandwich method, a competition method, a coagulation method (immunonephelometry or immunonephelometry), immunochromatography, capillary electrophoresis, western blotting, and a surface plasmon resonance method (SPR method), where a coagulation method is preferable, and a latex agglutination method is more preferable. In addition, regarding the measurement principle, any one of a homogenous method or a heterogeneous method may be used. The measuring method in the immunological measuring method is not particularly limited, and examples thereof include enzyme-linked immunosorbent assay (ELISA), enzyme immunoassay (EIA), radioimmunoassay (RIA), fluorescence enzyme immunoassay (FEIA), fluoroimmunoassay (FIA), chemiluminescence enzyme immunoassay (CLEIA), chemiluminescence immunoassay (CLIA), and electrochemical luminescence immunoassay (ECLIA).

### (Reagent kit)

The reagent kit according to the embodiment of the present invention is a reagent kit for measuring an endocrine protein, which contains the composition according to the embodiment of the present invention or the aqueous solution according to the embodiment of the present invention.

The reagent kit according to the embodiment of the present invention preferably includes the composition according to the embodiment of the present invention or the aqueous solution according to the embodiment of the present invention as a standard sample.

Specific examples of the reagent kit according to the embodiment of the present invention include a reagent kit in which the composition according to the embodiment of the present invention or the aqueous solution according to the embodiment of the present invention is contained in a container.

In the reagent kit according to the embodiment of the present invention, each component constituting the composition according to the embodiment of the present invention or the aqueous solution according to the embodiment of the present invention may be contained in a container or may be contained in an independent container.

Examples of the material of the container include glass and plastic. These preferred aspects are as described in the above-described composition.

The reagent kit according to the embodiment of the present invention may further include a reagent for dissolving the composition according to the embodiment of the present invention, a reagent for diluting the aqueous solution according to the embodiment of the present invention, a reagent or a container for measuring the amount of the endocrine protein, or the like.

Examples of the reagent for dissolving the composition according to the embodiment of the present invention or the reagent for diluting the aqueous solution according to the embodiment of the present invention include water, a buffer agent, a preservative, an excipient, a reaction accelerator, and the like. The kit according to the embodiment of the present invention may include these as separate reagents, or may include a mixture or solution of two or more of these.

Examples of the reagent for measuring the amount of the endocrine protein include an antibody, a reaction buffer solution, and a detection reagent, and the reagent can include, for example, a reagent used for measurement by a known immunological method without particular limitation.

The antibody may include, for example, a set of an antibody (primary antibody) against an endocrine protein and an antibody (secondary antibody) against the primary antibody. In addition, for example, for the reason of easily recovering a complex of an antibody and an antigen, such as a complex of a primary antibody and an antigen (endocrine protein), at least one of the primary antibody or the secondary antibody may be carried on a carrier such as resin beads or magnetic beads.

The primary antibody or the secondary antibody may be labeled with a known label for detection.

As the reaction buffer solution, a buffer solution known in the field of measurement by immunological methods can be included in the kit without particular limitation as a buffer solution used in the reaction between the antigen and the antibody.

As the detection reagent, a reagent known in the field of measurement by immunological methods can be included in the kit without particular limitation depending on the labeling of the antibody. For example, a reagent containing hydrogen peroxide and luminol can be used in a case where the antibody label is a peroxidase label. These reagents may be included in the kit separately into two or more reagents, for example, a reagent including hydrogen peroxide and a reagent including luminol, for the purpose of preventing the reaction from proceeding in the reagent.

The reagent kit according to the embodiment of the present invention can further include a user manual and the like.

### Examples

Hereinafter, the present invention will be described in detail using examples. Materials, using amounts, proportions, treatment details, treatment content, and the like shown in the following examples can be appropriately changed without departing from the gist of the present invention. Accordingly, the scope of the present invention is not limited to the following specific examples. Unless otherwise specified, "parts" and "%" are based on mass.

### (Comparative Example 1)

### 1. Production of endocrine protein solution (LH)

10.46 g (50 mM) of bis(2-hydroxyethyl)iminotris(hydroxymethyl)methane (manufactured by FUJIFILM Wako Pure Chemical Corporation), 1.7 mL (0.17%) of 5N-HCl (manufactured by FUJIFILM Wako Pure Chemical Corporation), and 20 g (2.0%) of bovine serum albumin (manufactured by Sigma-Aldrich Japan Co., Ltd.) were dissolved in 1,000 mL of purified water to produce a solvent for dissolving an endocrine protein.

Commercially available Luteinizing Hormone (manufactured by Meridien Japan Co., Ltd.) was dissolved in 10 mL of the solvent for dissolving an endocrine protein to prepare a high-unit LH solution at 10,000 mIU/mL. 3.0 mL of the high-unit LH solution was added to 300 mL of the solvent for dissolving an endocrine protein to produce an endocrine protein solution (LH) having an LH concentration of 100 mIU/mL.

### 2. Freeze-drying treatment

1 mL of the endocrine protein solution (LH) prepared in 1. described above was dispensed into a glass bottle, and subjected to a freeze-drying treatment using a vacuum freeze dryer.

### 3. Transfer of endocrine protein solution (LH) between containers (glass bottle + rubber stopper)

The freeze-dried product prepared in 2. described above was hydrated with 1 mL of purified water. The obtained aqueous solution was mixed by inverting 20 times in a state sealed with a rubber stopper. 800 µL of the solution was taken out from the endocrine protein solution (LH) after the mixing by inversion and transferred to a second glass container. The second glass container was mixed by inverting 20 times in a state sealed with a rubber stopper, and 600 µL of the solution was transferred to a third glass container. The third glass container was mixed by inverting 20 times in a state sealed with a rubber stopper, and 400 µL of the solution was transferred to a fourth glass container. The fourth glass container was mixed by inverting 20 times in a state sealed with a rubber stopper, and 200 µL of the solution was transferred to a fifth glass container.

### 4. Measurement of solution after transfer using Accuraseed

The concentrations of LH contained in the first, third, and fifth solutions of the glass container transferred in 3. described above and the solution before the transfer were measured using an automated chemiluminescent enzyme immunoassay analyzer Accuraseed (manufactured by FUJIFILM Wako Pure Chemical Corporation). The luminescence intensity of each solution was determined, the luminescence intensity of the solution before the transfer was set to 100%, and the luminescence intensity ratio was calculated. The results are shown in Table 1. A measuring method with Accuraseed will be described below.

### 5. Measuring method with Accuraseed (LH)

### (1) Preparation of reagent

A constitutional reagent required for the measurement was prepared using the following reagent raw materials.

MES (manufactured by Dojindo Chemical Co., Ltd.), sodium chloride (manufactured by FUJIFILM Wako Pure Chemical Corporation), BCN300S (manufactured by Nitta Gelatin Inc.), BLOCK ACE (manufactured by KAC Co., Ltd.), BSA (manufactured by Sigma-Aldrich Japan), sodium orthovanadate (manufactured by Sigma-Aldrich Japan), luminol sodium salt (manufactured by Sigma-Aldrich Japan), phosphoric acid (manufactured by FUJIFILM Wako Pure Chemical Corporation), EDTA-2Na (manufactured by Dojindo Chemical Co., Ltd.), hydrogen peroxide (manufactured by FUJIFILM Wako Pure Chemical Corporation), and dipotassium hydrogen phosphate (manufactured by FUJIFILM Wako Pure Chemical Corporation).

### (i) "First reagent" antibody-immobilized magnetic particle reagent

MAGRAPID MGP-010T (manufactured by Sanyo Chemical Industries, Ltd.) was sequentially reacted with 3-aminopropyltriethoxysilane (manufactured by Tokyo Chemical Industry Co., Ltd.) and succinic anhydride (manufactured by FUJIFILM Wako Pure Chemical Corporation), and then magnetic particles were collected with a neodymium magnet and the supernatant was removed to obtain magnetic particles having a carboxy group. Next, after activating the carboxyl groups using commercially available N-hydroxysuccinimide (manufactured by FUJIFILM Wako Pure Chemical Corporation) and WSC (manufactured by Dainippon Sumitomo Pharma Co., Ltd.), MAB<LH>M-2.406 IgG (manufactured by Roche Diagnostics K.K.) was reacted at 26°C for 12 to 16 hours. The magnetic particles were collected with a neodymium magnet, and the supernatant was removed to prepare antibody-immobilized magnetic particles. A first reagent having the following composition was then prepared.

"First reagent": 0.5 mg/mL of anti-LH antibody-immobilized magnetic particles, 50 mM MES (pH 5.5), 500 mM sodium chloride, and 3.0% BCN300S

### (ii) "Second reagent" reaction buffer solution

"Second reagent": 50 mM MOPS (pH 7.5), 500 mM sodium chloride, and 0.4% BLOCK ACE

### (iii) "Third reagent" reaction buffer solution

As an anti-LH antibody, commercially available MAB<LH>M-11412 IgG (manufactured by Roche Diagnostics K.K.) was digested with pepsin, and then F(ab')₂ was separated using a column (diameter: 1.5 cm × length: approximately 95 cm) filled with Sephacryl S-200HR (manufactured by Cytiva). The obtained F(ab')₂ was reduced with cysteamine hydrochloride (manufactured by Sigma-Aldrich Japan Co., Ltd.), and then Fab' was separated using a column (diameter: 1.5 cm × length: approximately 40 cm) filled with G-25 Superfine (manufactured by Cytiva). On the other hand, POD was maleimidated with a maleimidation reagent Sulfo-KMUS (manufactured by Dojindo Chemical Co., Ltd.), and the reaction mixture was subjected to a Sephadex G-25 column to remove unreacted Sulfo-KMUS, thereby obtaining maleimidated POD. The prepared Fab' was mixed with the maleimidated POD, and the mixture was separated using a Sephacryl S-100HR column to prepare a POD-labeled anti-LH antibody. Using this, a third reagent having the following composition was prepared.

"Third reagent": 1 to 100 nmol/L POD-labeled anti-LH antibody, 50 mM MES (pH 6.5), 150 mM sodium chloride, and 2.0% BSA

### (iV) "Fourth reagent"

"Fourth reagent": 1.0 mM sodium orthovanadate (pH 8.75), 150 mM sodium chloride, and 2 mM luminol sodium salt

### (V) "Fifth reagent"

"Fifth reagent": 68 µL/L phosphoric acid, 4 mM EDTA-2Na, and 335 µL/L hydrogen peroxide

### (2) Measurement of luminescence intensity

Using the first to fifth reagents described above, the luminescence intensities of the solutions were measured by the Accuraseed according to the following procedure. As the washing solution, an Accuraseed B/F separation solution (manufactured by FUJIFILM Wako Pure Chemical Corporation) was used.

50 µL of the first reagent added to the reaction cuvette was heated at 67°C for 20 seconds while magnetic particles were collected using a neodymium magnet, and the supernatant was removed. Subsequently, 50 µL of the second reagent and 25 µL of the measurement sample (endocrine protein solution (LH)) were added thereto, and the mixture was stirred and heated at 37°C for 3 minutes. After completion of the heating, the magnetic particles were collected using a neodymium magnet, a reagent other than the magnetic particles was removed, and the magnetic particles were washed three times with a washing solution. Subsequently, 50 µL of a third reagent was added thereto, the mixture was heated at 37°C for 3 minutes. After completion of the heating, the magnetic particles were collected using a neodymium magnet, a reagent other than the magnetic particles was removed, and the magnetic particles were washed three times with a washing solution. After completion of the washing, 100 µL of a fourth reagent and 100 µL of a fifth reagent were added, reacted at 37°C for 20 seconds, and then the luminescence intensity was measured.

### (Comparative Example 2)

### 1. Addition of fluorine-containing surfactant to endocrine protein solution (LH)

6.2 mg (final concentration: 0.02%) of Ftergent 250 (manufactured by NEOS COMPANY LIMITED) was added to 30 mL of the endocrine protein solution (LH) produced in Comparative Example 1.

### 2. Freeze-drying treatment

1 mL of the endocrine protein solution (LH) prepared in 1. described above was dispensed into a 1 mL glass bottle, and subjected to a freeze-drying treatment using a vacuum freeze dryer.

### 3. Transfer of endocrine protein solution (LH) between containers (glass bottle + rubber stopper)

The freeze-dried product prepared in 2. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1.

### (Comparative Example 3)

### 1. Addition of collagen peptide to endocrine protein solution (LH)

0.31 g (final concentration: 1.0%) of Nippi peptide AFC (manufactured by Nippi Inc.), which is a collagen peptide, was added to 30 mL of the endocrine protein solution (LH) produced in Comparative Example 1.

### 2. Freeze-drying treatment

1 mL of the endocrine protein solution (LH) prepared in 1. described above was dispensed into a 1 mL glass bottle, and subjected to a freeze-drying treatment using a vacuum freeze dryer.

### 3. Transfer of endocrine protein solution (LH) between containers (glass bottle + rubber stopper)

The freeze-dried product prepared in 2. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1.

### (Comparative Example 4)

### 1. Addition of milk protein to endocrine protein solution (LH)

0.12 g (final concentration: 0.4%) of Powder BLOCK ACE (manufactured by KAC Co., Ltd.), which is a milk protein, was added to 30 mL of the endocrine protein solution (LH) produced in Comparative Example 1.

### 2. Freeze-drying treatment

1 mL of the endocrine protein solution (LH) prepared in 1. described above was dispensed into a 1 mL glass bottle, and subjected to a freeze-drying treatment using a vacuum freeze dryer.

### 3. Transfer of endocrine protein solution (LH) between containers (glass bottle + rubber stopper)

The freeze-dried product prepared in 2. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1.

### (Example 1)

### 1. Addition of fluorine-containing surfactant and collagen peptide to endocrine protein solution (LH)

6.2 mg (final concentration: 0.02%) of Ftergent 250 (manufactured by NEOS COMPANY LIMITED) and 0.31 g (final concentration: 1.0%) of Nippi peptide AFC (manufactured by Nippi Inc.), which is a collagen peptide, was added to 30 mL of the endocrine protein solution (LH) produced in Comparative Example 1.

### 2. Freeze-drying treatment

1 mL of the endocrine protein solution (LH) prepared in 1. described above was dispensed into a 1 mL glass bottle, and subjected to a freeze-drying treatment using a vacuum freeze dryer.

### 3. Transfer of endocrine protein solution (LH) between containers (glass bottle + rubber stopper)

The freeze-dried product prepared in 2. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1.

### (Example 2)

### 1. Addition of fluorine-containing surfactant and milk protein to endocrine protein solution (LH)

6.2 mg (final concentration: 0.02%) of Ftergent 250 (manufactured by NEOS COMPANY LIMITED) and 0.12 g (final concentration: 0.4%) of Powder BLOCK ACE (manufactured by KAC Co., Ltd.), which is a milk protein, were added to 30 mL of the endocrine protein solution (LH) produced in Comparative Example 1.

### 2. Freeze-drying treatment

1 mL of the endocrine protein solution (LH) prepared in 1. described above was dispensed into a 1 mL glass bottle, and subjected to a freeze-drying treatment using a vacuum freeze dryer.

### 3. Transfer of endocrine protein solution (LH) between containers (glass bottle + rubber stopper)

The freeze-dried product prepared in 2. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1.

### (Example 3)

### 1. Addition of collagen peptide and milk protein to endocrine protein solution (LH)

0.31 g (final concentration: 1.0%) of Nippi peptide AFC (manufactured by Nippi Inc.), which is a collagen peptide, and 0.12 g (final concentration: 0.4%) of Powder Block Ace (manufactured by KAC. Co., Ltd.), which is a milk protein, were added to 30 mL of the endocrine protein solution (LH) manufactured in Comparative Example 1.

### 2. Freeze-drying treatment

1 mL of the endocrine protein solution (LH) prepared in 1. described above was dispensed into a 1 mL glass bottle, and subjected to a freeze-drying treatment using a vacuum freeze dryer.

### 3. Transfer of endocrine protein solution (LH) between containers (glass bottle + rubber stopper)

The freeze-dried product prepared in 2. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1.

### (Example 4)

### 1. Addition of fluorine-containing surfactant, collagen peptide, and milk protein to endocrine protein solution (LH)

6.2 mg (final concentration: 0.02%) of Ftergent 250 (manufactured by NEOS COMPANY LIMITED), 0.31 g (final concentration: 1.0%) of Nippi peptide AFC (manufactured by Nippi Inc.), which is a collagen peptide, and 0.12 g (final concentration: 0.4%) of Powder Block Ace (manufactured by KAC Co., Ltd.), which is a milk protein, were added to 30 mL of the endocrine protein solution (LH) produced in Comparative Example 1.

### 2. Freeze-drying treatment

1 mL of the endocrine protein solution (LH) prepared in 1. described above was dispensed into a 1 mL glass bottle, and subjected to a freeze-drying treatment using a vacuum freeze dryer.

### 3. Transfer of endocrine protein solution (LH) between containers (glass bottle + rubber stopper)

The freeze-dried product prepared in 2. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1.

### (Example 5)

### 1. Addition of fluorine-containing surfactant and milk protein (Casein) to endocrine protein solution (LH)

6.2 mg (final concentration: 0.02%) of Ftergent 250 (manufactured by NEOS COMPANY LIMITED) and 0.12 g (final concentration: 0.4%) of casein (manufactured by FUJIFILM Wako Pure Chemical Corporation), which is a milk protein, was added to 30 mL of the endocrine protein solution (LH) produced in Comparative Example 1.

### 2. Freeze-drying treatment

1 mL of the endocrine protein solution (LH) prepared in 1. described above was dispensed into a 1 mL glass bottle, and subjected to a freeze-drying treatment using a vacuum freeze dryer.

### 3. Transfer of endocrine protein solution (LH) between containers (glass bottle + rubber stopper)

The freeze-dried product prepared in 2. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1.

### (Example 6)

### 1. Production of endocrine protein solution (prolactin)

Commercially available purified human prolactin (manufactured by Aalto Bio Reagents Ltd.) was dissolved in 10 mL of the solvent for dissolving an endocrine protein produced in Comparative Example 1 to prepare a high-unit prolactin solution at 300 ng/mL. In addition, 3.0 mL of a 300 ng/mL prolactin solution was added to 300 mL of the solvent for dissolving endocrine protein produced in Comparative Example 1 to produce an endocrine protein solution (prolactin) having a prolactin concentration of 3.0 ng/mL.

### 2. Addition of collagen peptide and milk protein to endocrine protein solution (prolactin)

0.31 g (final concentration: 1.0%) of Nippi peptide AFC (manufactured by Nippi Inc.), which is a collagen peptide, and 0.12 g (final concentration: 0.4%) of Powder BLOCK ACE (manufactured by KAC Co., Ltd.), which is a milk protein, were added to an endocrine protein solution (prolactin).

### 3. Freeze-drying treatment

The endocrine protein solution (prolactin) prepared in 2. described above was dispensed into a 1 mL glass bottle, and subjected to a freeze-drying treatment using a vacuum freeze dryer.

### 4. Transfer of endocrine protein solution (prolactin) between containers (glass bottle + rubber stopper)

The freeze-dried product prepared in 3. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1. A measuring method with Accuraseed will be described below.

### 5. Measuring method with Accuraseed (prolactin)

The measurement was conducted by the same method as the method in 5. Measuring method with Accuraseed (LH) of Comparative Example 1, except for the following.

Commercially available MERMAF10-111 Antibody to Prolactin (manufactured by Meridien Japan Co., Ltd.) was used instead of a commercially available MAB<LH>M-2.406 IgG (manufactured by Roche Diagnostics K.K.). A commercially available MERMAF10-245 Antibody to Prolactin (manufactured by Meridien Japan Co., Ltd.) was used instead of a commercially available MAB<LH>M-11412 IgG (manufactured by Roche Diagnostics K.K.) to obtain a POD-labeled anti-prolactin antibody. In addition, a POD-labeled anti-prolactin antibody was used instead of the POD-labeled anti-LH antibody.

### (Example 7)

### 1. Production of endocrine protein solution (hCG)

Commercially available human chorionic gonadotropin (manufactured by Meridien Japan Co., Ltd.) was dissolved in 10 mL of the solvent for dissolving an endocrine protein dissolving produced in Comparative Example 1 to prepare a high-unit hCG solution at 1,000 mIU/mL. 3.0 mL of a 1,000 mIU/mL hCG solution was added to 300 mL of the solvent for dissolving an endocrine protein, produced in Comparative Example 1, to produce an endocrine protein solution (hCG) having an hCG concentration of 10 mIU/mL.

### 2. Addition of collagen peptide and milk protein to endocrine protein solution (hCG)

0.31 g (final concentration: 1.0%) of Nippi peptide AFC (manufactured by Nippi Inc.), which is a collagen peptide, and 0.12 g (final concentration: 0.4%) of Powder BLOCK ACE (manufactured by KAC Co., Ltd.), which is a milk protein, were added to an endocrine protein solution (hCG).

### 3. Freeze-drying treatment

The endocrine protein solution (hCG) prepared in 2. described above was dispensed into a 1 mL glass bottle, and subjected to a freeze-drying treatment using a vacuum freeze dryer.

### 4. Transfer of endocrine protein solution (hCG) between containers (glass bottles+ rubber stopper)

The freeze-dried product prepared in 3. described above was hydrated with 1 mL of purified water. The obtained aqueous solution was added to a glass container in the same manner as in Comparative Example 1, and in a state sealed with a rubber stopper, after mixing by inversion, transfer of the solution between glass containers five times. The luminescence intensity was measured with an Accuraseed, and the luminescence intensity ratio was calculated with the luminescence intensity of the solution before the transfer set to 100%. The results are shown in Table 1. A measuring method with Accuraseed will be described below.

### 5. Measuring method with Accuraseed (hCG)

The measurement was conducted by the same method as the method in 5. Measuring method with Accuraseed (LH) of Comparative Example 1, except for the following.

Commercially available MAb to hCG beta (manufactured by Meridien Japan Co., Ltd.) was used instead of a commercially available MAB<LH>M-2.406 IgG (manufactured by Roche Diagnostics K.K.). A commercially available MAB<HCG>M-INN22 IgG (manufactured by Roche Diagnostics K.K.) was used instead of a commercially available MAB<LH>M-11412 IgG (manufactured by Roche Diagnostics K.K.) to obtain a POD-labeled anti-hGC antibody. In addition, a POD-labeled anti-hCG antibody was used instead of the POD-labeled anti-LH antibody.

### (Comparative Example 5)

The transfer of the endocrine protein solution (LH) produced and hydrated in Comparative Example 1 between the containers was performed and the luminescence intensity was measured and compared using Accuraseed by the same method as in Comparative Example 1 except that an eppendorf tube made of PP material was used instead of the glass container with a rubber stopper. The results are shown in Table 1.

### (Example 8)

The transfer to an eppendorf tube made of PP material, and the luminescence intensity was measured and compared using Accuraseed in the same manner as Comparative Example 5, except that the freeze-drying treatment and the hydration were performed using a solution obtained by adding 0.31 g (final concentration: 1.0%) of Nippi peptide AFC (manufactured by Nippi Inc.), which is a collagen peptide, and 0.12 g (final concentration: 0.4%) of Powder BLOCK ACE (manufactured by KAC Co., Ltd.), which is a milk protein, to 30 mL of endocrine protein solution (LH) produced in Comparative Example 1 by the same method as in Example 3, instead of the endocrine protein solution (LH). The results are shown in Table 1.

In Comparative Example 1, the luminescence intensity of the transferred fifth solution was 76% with respect to the luminescence intensity of the solution before the transfer, and the adsorption of LH to the glass and the rubber stopper was confirmed. From the results of Comparative Examples 2 to 4, in the case of the single addition of A) the fluorine-containing surfactant, B) the collagen peptide, or C) the milk protein, the adsorption of LH occurred, and the decrease in the luminescence intensity after the transfer could not be suppressed.

However, from the results of Examples 1 to 3, it was found that by allowing at least two components selected from A) a fluorine-containing surfactant, B) a collagen peptide, and C) a milk protein to coexist, the decrease in the luminescence intensity after transfer is suppressed, and the effect of preventing adsorption to glass and a rubber stopper is exhibited. From the results of Example 4, it was found that the same effect was exhibited even in a case where three components of A) fluorine-containing surfactant, B) collagen peptide, and C) milk protein coexisted. In addition, from the results of Example 5, it was found that the same effect was exhibited even in a case where casein was used instead of Powder BLOCK ACE as C) the milk protein. From the results of Examples 6 and 7, it was found that, similarly to LH, also for prolactin and hCG, the effect of preventing adsorption was exhibited by coexisting at least two components selected from A) a fluorine-containing surfactant, B) a collagen peptide, and C) a milk protein.

In addition, from the results of Comparative Example 5 and Example 8, it was found that this effect was exhibited not only for the glass + rubber stopper but also for a container made of PP material.

**[Table 1]**

| | Endocrine protein | Fluorine-containing surfactant | Collagen peptide | Milk protein | Container | Luminescence intensity ratio to luminescence intensity before transfer First bottle | Luminescence intensity ratio to luminescence intensity before transfer Third bottle | Luminescence intensity ratio to luminescence intensity before transfer Fifth bottle |
|---|---|---|---|---|---|---|---|---|
| Comparative Example 1 | LH | Not use | Not use | Not use | Glass | 95% | 91% | 76% |
| Comparative Example 2 | LH | Use | Not use | Not use | Glass | 93% | 87% | 79% |
| Comparative Example 3 | LH | Not use | Use | Not use | Glass | 92% | 82% | 72% |
| Comparative Example 4 | LH | Not use | Not use | Use | Glass | 96% | 91% | 85% |
| Example 1 | LH | Use | Use | Not use | Glass | 100% | 100% | 99% |
| Example 2 | LH | Use | Not use | Use | Glass | 100% | 99% | 100% |
| Example 3 | LH | Not use | Use | Use | Glass | 98% | 97% | 97% |
| Example 4 | LH | Use | Use | Use | Glass | 100% | 100% | 98% |
| Example 5 | LH | Use | Not use | Use | Glass | 100% | 100% | 100% |
| Example 6 | PRL | Not use | Use | Use | Glass | 100% | 96% | 98% |
| Example 7 | hCG | Not use | Use | Use | Glass | 99% | 100% | 98% |
| Comparative Example 5 | LH | Not use | Not use | Not use | Plastic | 98% | 95% | 89% |
| Example 8 | LH | Not use | Use | Use | Plastic | 98% | 100% | 100% |

## Claims

1. A composition comprising:
an endocrine protein; and
at least two compounds selected from the group consisting of a fluorine-containing surfactant, a collagen peptide, and a milk protein.

2. The composition according to claim 1,
wherein the composition is a standard sample for measuring an amount of an endocrine protein.

3. The composition according to claim 1,
wherein the composition is contained in a container.

4. The composition according to claim 3,
wherein a material of the container is glass or plastic.

5. The composition according to claim 1,
wherein a content of the fluorine-containing surfactant is 0.1% to 1% by mass with respect to a total solid content of the composition.

6. The composition according to claim 1,
wherein a content of the collagen peptide is 1% to 30% by mass with respect to a total solid content of the composition.

7. The composition according to claim 1,
wherein a content of the milk protein is 0.1% to 10% by mass with respect to a total solid content of the composition.

8. The composition according to claim 1,
wherein the endocrine protein is luteinizing hormone, prolactin, or human chorionic gonadotropin.

9. An aqueous solution of an endocrine protein, comprising:
water;
an endocrine protein; and
at least two compounds selected from the group consisting of a fluorine-containing surfactant, a collagen peptide, and a milk protein.

10. The aqueous solution according to claim 9,
wherein the aqueous solution is a standard sample for measuring an amount of an endocrine protein.

11. The aqueous solution according to claim 9,
wherein the aqueous solution is contained in a container.

12. The aqueous solution according to claim 11,
wherein a material of the container is glass or plastic.

13. The aqueous solution according to claim 9,
wherein a content of the fluorine-containing surfactant is 0.1% to 1% by mass with respect to a total solid content of the aqueous solution.

14. The aqueous solution according to claim 9,
wherein a content of the collagen peptide is 1% to 30% by mass with respect to a total solid content of the aqueous solution.

15. The aqueous solution according to claim 9,
wherein a content of the milk protein is 0.1% to 10% by mass with respect to a total solid content of the aqueous solution.

16. The aqueous solution according to claim 9,
wherein the endocrine protein is luteinizing hormone, prolactin, or human chorionic gonadotropin.

17. An adsorption inhibitor for an endocrine protein, comprising:
at least two compounds selected from the group consisting of a fluorine-containing surfactant, a collagen peptide, and a milk protein.

18. A method for suppressing adsorption of an endocrine protein, the method comprising:
bringing a glass or plastic member into contact with an endocrine protein in presence of water and at least two compounds selected from the group consisting of a fluorine-containing surfactant, a collagen peptide, and a milk protein.

19. A method for suppressing adsorption of an endocrine protein, comprising:
bringing a glass or plastic member into contact with an aqueous solution containing water, an endocrine protein, and at least two compounds selected from the group consisting of a fluorine-containing surfactant, a collagen peptide, and a milk protein.

20. A method for measuring an amount of an endocrine protein, the method comprising:
using, as a standard sample, the composition according to any one of claims 1 to 8 or the aqueous solution according to any one of claims 9 to 16.

21. A reagent kit for measuring an endocrine protein, the reagent kit comprising:
the composition according to any one of claims 1 to 8 or the aqueous solution according to any one of claims 9 to 16.
